Europäisches Patentamt

European Patent Office

Office européen des brevets

(11) Publication number: **0 099 253**
**B 1**

# EUROPEAN PATENT SPECIFICATION

(45) Date of publication of patent specification: **30.12.86**

(51) Int. Cl.⁴: **A 61 B 5/04, A 61 N 1/04**

(21) Application number: **83303987.8**

(22) Date of filing: **08.07.83**

(54) Micro electrodes and the production thereof.

(30) Priority: **08.07.82 JP 119035/82**

(43) Date of publication of application:
**25.01.84 Bulletin 84/04**

(45) Publication of the grant of the patent:
**30.12.86 Bulletin 86/52**

(84) Designated Contracting States:
**CH DE FR IT LI**

(56) References cited:
**EP-A-0 024 963**
**US-A-3 540 434**
**IEEE TRANSACTIONS ON BIOMEDICAL
ENGINEERING, vol. BME-20, no. 4, July 1973,
pages 260-269, New York, US; S.R.
GOLDSTEIN et al.: "Mechanical factors in the
design of chronic recording intracortical
microelectrodes"**
**MEDICAL & BIOLOGICAL ENGINEERING &
COMPUTING, vol. 15, no. 3, May 1977, pages
327-332, Stevenage, US; Y.J. KINGMA:
"Measurements on glassy-carbon electrodes in
saline"**

(73) Proprietor: **SHIONOGI & CO., LTD.**
**12, Dosho-machi 3-chome Higashi-ku
Osaka 541 (JP)**

(72) Inventor: **Miyazaki, Hiroshi**
**80-24, Nimyo-cho
Nara-shi Nara (JP)**
Inventor: **Ikeda, Masato**
**21-14, 3-chome Shikanodai-Nishi
Ikoma-shi Nara (JP)**

(74) Representative: **Bizley, Richard Edward et al
BOULT, WADE & TENNANT 27 Furnival Street
London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

# Description

The present invention relates to micro electrodes and the production and use thereof.

Micro electrodes may be designed to be directly inserted deeply into a living body. In addition to the passive function of simply picking up biological signals from such a position, they may have the active function of supplying the implanted position with an external electric current to effect an electrochemical reaction. Micro electrodes having such an active function are designated as "micro working electrodes" and are useful in analyzing by means of voltammetry minute quantities of substances produced at the implantation position in the living body as a result of such electrochemical reactions. Measurements made by such electrodes can be processed in an external appliance to give valuable information on the bio-electrochemical phenomena of the living body.

Amongst other uses, a micro working electrode can also be placed in the fluid path in a liquid chromatography system with a view to detecting minute or trace amounts of substances present in the running liquid.

Since such micro working electrodes are to be inserted in and fixed at specified positions having extremely small dimensions, for instance, around the nuclei of the cranial nerves of the rat, the whole electrode structure has to be designed taking account of many restrictive conditions.

Such electrodes are usually structured to have core components, i.e. a conductive filament and an outer lead wire, and supporting components, i.e., an envelope of a physiologically inert material, for instance, glass, and a filler of, for instance, a resinous material, for embedding the core components in the envelope.

The exposed segment of the conductive filament, which projects from the tip of the envelope and serves as the site of the electrochemical reaction, should be small in size in order to give the electrode high selectivity with respect to the spot whereat it contacts the living body, and the filament itself should be as thin as possible. As conductive filaments thin metal wires have been used hitherto. However, metal wires as thin as 100 µm have insufficient mechanical strength. In addition, some metals are inherently not suited for the purpose on account of their electrochemical properties. Moreover, micro working electrodes are required to have a residual current as small as possible and thus the insulation around the exposed segment is important.

Recently the use of carbon fibers for this purpose has been proposed, and a method of electrode construction and use has been reported (see Jean-Luc Ponchon et al., ANALYTICAL CHEMISTRY, 51(9), 1483—1486, 1979).

Although the carbon fibers are excellent in their mechanical properties, e.g., deflective strength, and may be finished to very thin monofilaments, they are unexpectedly difficult to handle in assembling micro electrodes. In cases where a tapering glass capillary tube is selected as a suitable envelope, the manipulation involved in threading a very thin carbon monofilament into the needle end of the capillary tube has proved much more difficult than expected.

According to the method disclosed in the above-mentioned report, a glass tube is worked by drawing using a "pipet puller" to obtain a tip diameter of a few µm and by cutting the tube into a capillary (length, 10 to 30 mm). A carbon fiber (length, 20 to 40 mm; diameter 8 µm) is threaded into the capillary until it is blocked by the fringed tip. The glass capillary is cut at the level where the fiber is blocked, thus enabling the fiber to be pushed a few mm through the capillary. This method minimizes the interstitial space between the capillary and the carbon fiber. The cavity of the capillary is then packed with a conductive paste (polyester resin containing graphite powder), first by inverting the capillary into a mass of the conductive paste to fill a part of the cavity with the paste and then by forcing the filled paste into the tip of the capillary tube. At the tip of the capillary, the resin is separated from the graphite powder (about 1 µm in diameter) to ensure insulation. Connection of the carbon fiber with an outer lead wire is made simply by pushing the wire as far as possible into the capillary cavity filled with the paste. More importantly, the carbon monofilament is connected with the outer lead wire as a result of their being embedded in a common conductive resin.

As will be indicated later, an electrode prepared by such a method as described above is not satisfactory in its performance and in its production.

There is thus a need for a simple and reliable micro working electrode structure which overcomes the above-mentioned disadvantages and for a production method which is suited for mass-producing the same in an easy and simple manner allowing high productivity. This invention is based upon the discovery that threading the carbon fiber into the capillary tube is made easy by filling the cavity of the capillary tube with a volatile solvent. By this means a direct connection of the carbon monofilament with the outer lead wire is also made possible and an insulating filler may be used in place of the conductive paste.

According to the present invention there is provided a micro electrode comprising a tapering capillary tube in which the thicker end preferably has an outside diameter of 2 mm or less, a conductive filament, preferably having an outside diameter of 50 µm or less, an outer lead wire and a filler packed into the cavity of the capillary tube, the conductive filament being directly connected with the outer lead wire in the cavity of the capillary tube and the filler being an insulating resin, the conductive filament and the outer lead wire being discrete from each other.

According to another aspect of the present invention there is provided a method for producing a micro electrode which includes the step of threading a conductive filament into a tapering glass capillary tube so that the tip of the filament projects from the needle end of the tube, at least part of the cavity of the capillary tube being filled with a volatile solvent during the threading step.

The invention also relates to the use of a micro electrode, as defined in appended independent claim 10.

The conductive filament is preferably carbon fiber and a single monofilament is usually used. However, two or more monofilaments may be used in a bundle to enlarge the effective surface area of the electrode and to improve sensitivity.

A metal wire may also be used as the conductive filament and can easily be connected with the lead wire by conventional means, for instance, soldering. The metal wire should however, have a deflective strength equivalent to or higher than that of a carbon fiber of the same diameter.

The conductive filament, e.g., a carbon monofilament or a bundle of monofilaments, is preferably connected directly with the outer lead wire by, for instance, gluing the former to the latter with a conductive resin containing carbon powder (usually available as a conductive paint or adhesive) prior to the threading step. The method of the present invention makes such direct connection and the structure incorporating the thus-connected body possible.

Since the conductive filament is directly connected with the outer lead wire and the conductive segment in the cavity of the capillary tube is strictly limited to the zone of connection between wire and filament, the remaining part of the cavity does not need to be conductive and is usually filled with an insulating resin in the electrode structure of the present invention. This structure ensures a highly reliable connection and a high degree of insulation as compared to conventional structures. In addition, since the size of a pinhole formed at the needle end of the capillary tube may vary widely (about 7—100 μm), productivity in terms of yield is sufficiently high.

The volatile solvent used in the production process can be, for example, selected from alcohols, e.g., ethanol and methanol, ketones, e.g., acetone and methyl ethyl ketone, ethers, e.g., diethyl ether and tetrahydrofuran, esters, e.g., ethyl acetate, and hydrocarbons, e.g., benzene and hexane. Water can also be used as the volatile solvent in certain cases. Alcohols, particularly ethanol, are preferred in view of ease and safety in operation and the short drying time required.

Since the capacity of the capillary is very small its entire space is usually filled with the volatile solvent, but only a part, which corresponds to the needly end of the cavity, is necessarily moistened.

The present invention will now be described and illustrated in more detail by referring to the attached drawings, in which:

Fig. 1 is a partly cut-out side view of a micro electrode embodying the present invention; and

Figs. 2 and 3 are similar schematic views illustrating the production process of the invention.

In the drawings reference numeral 1 represents a capillary tube, 2 is an outer lead wire with an insulator coating layer 22, 3 is a carbon fiber, 4 is a carbon powder-containing conductive resin layer, 5 is an insulator resin layer and 6 is a protective resin layer.

The tip 31 (length usually 0.1 to 0.5 mm) of the carbon monofilament 3 projects exposed from the needle end 11 of the capillary tube 1. The root segment 32 of the carbon monofilament 3 is glued to a connecting segment 21 of the outer lead wire 2, where its insulator coating layer 22 is removed, with the carbon powder-containing conductive resin layer 4. The opposite end of the lead wire 2 is exposed from the thicker end of the capillary tube for connection to outside appliances.

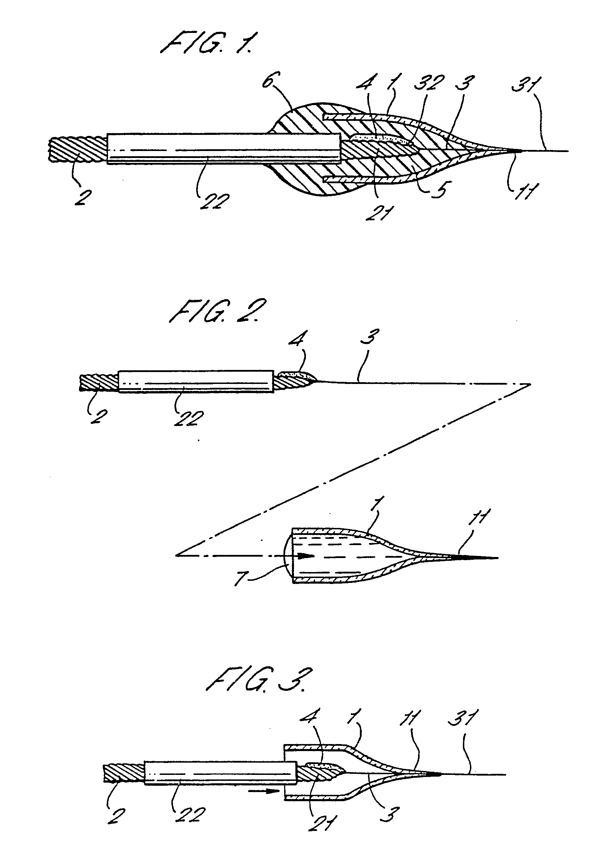

In producing the electrode, the carbon monofilament 3 is first glued on to the outer lead wire 2 to give a combined body. Separately, the tapering capillary tube 1 is prepared by working a micropipette (about 100 μL in capacity, 1.5 mm outside diameter and 1.1 mm inside diameter) by a pipet puller to form a needle end (about 8 μm to 200 μm inside diameter) and by cutting the micropipette at the pulled portion to give pieces of about 15 mm in length. Then the cavity is filled with a volatile solvent 7 (ethanol, about 10 μL), into which the combined body is introduced (as indicated by the dotted line — Fig. 2) so that the tip 31 to the carbon monofilament 3 is threaded into the needle end to project therefrom. If no such solvent is used and the cavity is left empty, introduction and threading of the carbon monofilament 3 is so difficult as to result in frequent breakage and sometimes is substantially impossible. After the completion of the threading step, the glued segments of the carbon monofilament 3 and the lead wire 2 are enclosed in the cavity of the capillary tube 1 (Fig. 3).

When the volatile solvent is completely removed from the cavity after a short drying time, an insulating resin 5 in a fluid state is poured into the cavity of the glass capillary tube 1 from its thicker end using a syringe along the direction indicated by an arrow Fig. 3 to embed the combined body. The fluid resin is evenly distributed up to the tip of the needle end 11 of the capillary tube 1 through capillary action but it will not leak from the interstice between the monofilament 3 and the encircling glass. The resin is preferably of low viscosity in its fluid state in order to take best advantage of the capillary action.

The thicker end of the capillary tube 1 is then protected with another insulator resin layer 6. As the resin for this purpose, the resin used in the aforesaid embedding can be used, although it is preferred to select one having more complete protection capability and a shorter hardening time.

In one example, TORAYCA T 300 or M 40, available from Toray Corporation, may be used as the carbon fiber, Dotite SH—3A (Epoxide resin base), available from Fujikura Kasei K.K., may be used as the conductive resin, Araldite AY 103/HY 956, available from Ciba-Geigy, may be used as the resin for embedding, and Hi-Super, available from Cemedain K.K., may be used as the protective coating layer.

The thus-obtained micro electrode is finished by cutting the tip 31 of the carbon monofilament 3 by 500 μm, and subjecting it to a conduction test and to electrolytic treatment (which is a cyclic anodic pre-treatment in dilute sulfuric acid).

The electrical resistance of the electrode is usually around 4 to 5 K Ohms, which is mainly attributable to the resistance of the carbon monofilament. The residual current is of the order of nA (nano ampere).

In a comparative experiment, an electrode of the same structure as that in the illustrated example is prepared by substituting Dotite SH—3A for Araldite AY 103/HY 956 as the resin for embedding the combined body (the solvent contained to excess in Dotite SH—3A having been extracted therefrom before use). The thus-obtained electrode showed a very large value for residual current (about 100 times that of the illustrated embodiment of the invention) and cannot be used in precision measurements.

Incidentally, since the electrical resistance of the electrode is mainly accountable as that of the carbon monofilament, the use of a plurality of monofilaments in a bundle will decrease the electrical resistance. This facilitates the adjustment of the electrode to a measuring appliance.

On the other hand, the electrode may be finished by cutting the tip of the monofilament up to 100 μm or shorter in order to improve selectivity for the spot where it is to be positioned. In an extreme case, the electrode may be in the form of a disk in a sense of electrical equivalence.

## Claims

1. A micro electrode comprising a tapering capillary tube (1) with a conductive filament (3) projecting therefrom and directly connected to an outer lead wire (2) at a position within the cavity of the capillary tube, the cavity of the capillary tube being packed with an insulating resin (5) and the conductive filament and the outer lead wire being discrete from each other.

2. A micro electrode as claimed in claim 1, wherein the wide end of the capillary tube (1) has an outside diameter of 2 mm or less.

3. A micro electrode as claimed in claim 1 or claim 2, wherein the conductive filament (3) has an outside diameter of 50 μm or less.

4. A micro electrode as claimed in any one of claims 1 to 3, wherein the conductive filament (3) is carbon fiber.

5. A micro electrode as claimed in any one of claims 1 to 4, wherein the conductive filament (3) is glued to the outer lead wire (2) with a conductive resin (4) containing carbon powder.

6. A method for producing a micro electrode including filling at least part of the cavity of a tapering capillary (1) tube with a volatile solvent (7) and threading a conductive filament (3) into the thus-filled capillary tube so that the tip of the filament projects from the thin end of the tapering capillary tube.

7. A method as claimed in claim 6, wherein the conductive filament (3) is carbon fiber.

8. A method as claimed in claim 6 or claim 7, wherein the conductive filament (3) is directly connected to a lead wire (2) prior to the threading step.

9. A method as claimed in claim 8, wherein the conductive filament (3) and lead wire (2) are glued together with a conductive resin (4) containing carbon powder.

10. The use of a micro electrode as claimed in any one of claims 1 to 5 or of a micro electrode which has been produced by a method as claimed in any one of claims 6 to 9 in detecting trace amounts of substances in fluids or in investigating the bio-electrical properties of a living body other than in diagnosis.

## Patentansprüche

1. Mikroelektrode, umfassend ein sich verjüngendes Kapillarrohr (1) mit einem aus diesem herausragenden, leitenden Draht (3), der unmittelbar mit einem äußeren Zuleitungsdraht (2) an einer innerhalb des Hohlraums des Kapillarrohrs liegenden Stelle verbunden ist, wobei der Hohlraum des Kapillarrohres mit einem Isolierharz (5) gefüllt ist und der leitende Draht und der äußere Zuleitungsdraht diskret einander gegenüberliegend ausgebildet sind.

2. Mikroelektrode nach Anspruch 1, bei welcher das breite Ende des Kapillarrohres (1) einen Außendurchmesser von 2 mm oder weniger aufweist.

3. Mikroelektrode nach Anspruch 1 oder 2, bei welcher der leitende Draht (3) einen Außendurchmesser von 50 μm oder weniger aufweist.

4. Mikroelektrode nach einem der Ansprüche 1 bis 3, bei welcher der leitende Draht (3) aus einer Kohlenstoffaser besteht.

5. Mikroelektrode nach einem der Ansprüche 1 bis 4, bei welcher der leitende Draht (3) mit dem äußeren Zuleitungsdraht (2) mit einem leitenden Harz (4), das ein Kohlenstoffpulver enthält, verklebt ist.

6. Verfahren zur Herstellung einer Mikroelektrode, einschließend das Füllen mindestens eines Teils des Hohlraums eines sich verjüngenden Kapillarrohres (1) mit einem flüchtigen Lösungsmittel (7) und Einführen eines leitenden Drahtes in das auf diese Weise gefüllte Kapillarrohr, so daß das vordere Ende des Drahtes vom dünnen Ende des sich verjüngenden Kapillarrohres nach außen vorsteht.

7. Verfahren nach Anspruch 6, bei welchem der Leitende Draht (3) eine Kohlenstoffaser ist.

8. Verfahren nach Anspruch 6 oder 7, bei welchem der leitende Draht (3) unmittelbar mit einem Zuleitungsdraht (2) verbunden wird, bevor der Einführungsschritt erfolgt.

9. Verfahren nach Anspruch 8, bei welchem der leitende Draht (3) und der Zuleitungsdraht (2) mit einem leitenden Harz (4), das Kohlenstoffpulver enthält, miteinander verklebt werden.

10. Verwendung einer Mikroelektrode nach einem der Ansprüche 1 bis 5 oder einer Mikroelektrode, die durch ein Verfahren nach einem der Ansprüche 6 bis 9 hergestellt wurde, zur Erfassung von Spurenmengen von in Fluiden befindlichen Substanzen oder zur Untersuchung bio-elektrochemischer Eigenschaften eines lebenden Körpers außerhalb eines diagnostischen Einsatzes.

**Revendications**

1. Micro-électrode comprenant un tube capillaire rétréci (1) avec un filament conducteur (3) qui en dépasse et est directement relié à un fil conducteur extérieur (2) en une position à l'intérieur de la cavité du tube capillaire, la cavité du tube capillaire étant bourrée d'une résine isolante (5) et le filament conducteur et le fil conducteur extérieur étant distincts l'une de l'autre.

2. Micro-électrode selon la revendication 1, dans laquelle l'extrémité large du tube capillaire (1) a un diamètre extérieur de 2 mm ou moins.

3. Micro-électrode selon la revendication 1 ou la revendication 2, dans laquelle le filament conducteur (3) a un diamètre extérieur de 50 µm ou moins.

4. Micro-électrode selon l'une quelconque des revendications 1 à 3, dans laquelle le filament conducteur (3) est une fibre de carbone.

5. Micro-électrode selon l'une quelconque des revendications 1 à 4, dans laquelle le filament conducteur (3) est collé au fil conducteur extérieur (2) avec une résine conductrice (4) contenant de la poudre de carbone.

6. Procédé de fabrication d'une micro-électrode, consistant à remplir d'un solvant volatif (7) au moins une partie de la cavité d'un tube capillaire rétréci (1) et à enfiler un filament conducteur (3) dans le tube capillaire ainsi rempli de sorte que le bout du filament dépasse de l'extrémité mince du tube capillaire rétréci.

7. Procédé selon la revendication 6, dans lequel le filament conducteur (3) est une fibre de carbone.

8. Procédé selon la revendication 6 ou la revendication 7, dans lequel on relie directement le filament conducteur (3) à un fil conducteur (2) avant l'étape d'enfilage.

9. Procédé selon la revendication 8, dans lequel on colle ensemble le filament conducteur (3) et le fil conducteur (2) avec une résine conductrice (4) contenant de la poudre de carbone.

10. Utilisation d'une micro-électrode selon l'une quelconque des revendications 1 à 5, ou d'une micro-électrode qui a été fabriquée par un procédé selon l'une quelconque des revendications 6 à 9, dans la détection de traces de substances dans des fluides ou dans l'étude des propriétés bio-électro-chimiques d'un organisme vivant, autrement que dans le diagnostic.

FIG. 1.

FIG. 2.

FIG. 3.